# EUROPÄISCHE PATENTANMELDUNG

(11) **EP 0 832 631 A2**
(43) Veröffentlichungstag der Anmeldung: **01.04.1998**
(21) Anmeldenummer: 97116770.5
(22) Anmeldetag: 26.09.1997
(51) Int. Cl.: A61F 13/58, A61F 13/62

(54) **Windelverschlussband und Windel mit einem derartigen Verschlussband**

(30) Priorität: 27.09.1996 DE 19639848; 24.10.1996 DE 19644230
(71) Anmelder: Koester GmbH & Co. KG, 96146 Altendorf (DE)
(72) Erfinder: Bräunig, Werner, D-97514 Dankenfeld (DE); Neugebauer, Robert, D-91077 Neunkirchen am Brand (DE)
(74) Vertreter: Castell, Klaus, Dr.

(57) **Zusammenfassung**

Das Windelverschlußband hat neben einem ersten Bereich, mit dem es an einer Windel befestigt ist, und einem zweiten Bereich, der lösbar mit der Windel verbindbar ist, einen dritten Bereich in der Nähe des zweiten Bereiches, der ein spaltbares Material aufweist, dessen eine Seite an der Windel befestigbar ist.

Dieses spaltbare Material hält das Windelverschlußband fest an der Windel und wird zur Benutzung der Windel durch Ziehen am Anfaßbereich so aufgespalten, daß zwei flauschige, weiche Flächen entstehen.

## Beschreibung

Die Erfindung betrifft ein Windelverschlußband mit einem ersten Bereich, der an einer Windel befestigbar ist und mit einem zweiten Bereich, der lösbar mit der Windel verbindbar ist.

Derartige Windelverschlußbänder werden dazu verwendet, die angelegte Windel so zu verschließen, daß sie am Körper anliegt. Hierzu ist ein erster Bereich des Windelverschlußbandes mit der Windel - vorzugsweise der hinteren Seite der Windel - fest verbindbar, während ein zweiter Bereich lösbar - vorzugsweise mit der vorderen Seite der Windel - verbindbar ist.

Unter Windel werden wegwerfbare Babywindeln, Windeln mit austauschbaren Einlagen sowie Windelsysteme für Inkontinente und alle anderen verschließbaren Windelsysteme verstanden.

Außerdem betrifft die Erfindung eine Windel mit einem derartigen Windelverschlußband.

Bekannte Windelverschlußbänder haben den Nachteil, daß das Windelverschlußband in dem Bereich zwischen dem ersten und dem zweiten Bereich des Bandes zur Faltenbildung neigt. Außerdem kann sich der zweite, lösbar mit der Windel verbindbare Teil des Windelverschlußbandes während des Schneidvorgangs in der Windelmaschine und nach dem Applizieren in der Windelmaschine von der Windel lösen und steht dann störend von der Windel ab.

Der Erfindung liegt daher die Aufgabe zugrunde, ein Windelverschlußband und eine Windel derart weiterzuentwickeln, daß eine problemlose Verarbeitung in der Windelmaschine gewährleistet ist.

Diese Aufgabe wird mit einem Windelverschlußband gelöst, bei dem ein in der Nähe des zweiten Bereiches angeordneter dritter Bereich ein spaltbares Material aufweist, dessen eine Seite an der Windel befestigbar ist.

Die Verwendung eines spaltbaren Materials in einem dritten Bereich bietet den großen Vorteil, daß das Windelverschlußband in diesem Bereich während der Verarbeitung und dem Transport der Windel durch die Windelmaschine fest mit der Windel in Verbindung gehalten werden kann. Bei der späteren Verwendung der Windel wird das spaltbare Material im dritten Bereich derart aufgespalten, daß eine Seite fest mit der Windel verbunden bleibt und die andere Seite einen Teil des Windelverschlußbandes bildet oder fest mit diesem verbunden ist.

Spaltbare Materialien bestehen aus gleichen oder unterschiedlichen Komponenten, die ohne klebrige Grenzfläche aneinanderhaften. Sie sind daher im getrennten Zustand unklebrig und haften trotzdem aneinander.

Als spaltbare Materialien eignen sich Papier, Folien und Nonwovens deren Cohäsion im Bereich von 0,1 - 10 N/inch, vorzugsweise 2,5 - 7 N/inch liegt. Als spaltbare Materialien eignen sich auch zwei- oder mehrschichtige Verbundstoffe wie z. B. PE/PP deren Verbundhaftung im Bereich von 0,1 - 7 N/inch liegt, vorzugsweise 0,4 - 2,5 N/inch.

Als Beispiele sind Polyethylen/Polypropylen, Polypropylen/Polypropylen oder Polyethylen/Polyethylensowie Polyethylen/Nonwoven, Polypropylen/Nonwoven, Polyethylen/Papier und Polypropylen/Papier oder PP/Nonwoven/PP oder HDPE/Nonwoven/PP zu nennen.

Zur Gruppe der Materialkombination Polyethylen/Polyethylen zählt das vorteilhafterweise verwendete MDPE. Dies ist ein Polyethylen mit mittlerer Dichte. Dieses Material wird mit E/VA (Ethylenvinylacetat-Copolymere) klebrig gemacht und als Antiblockiermittel wird Siliciumoxid (SiO₂) eingesetzt.

Derartige Doppelfolien werden vorzugsweise als Schlauch hergestellt. Ein durch Blasextrusion hergestellter Schlauch hat eine Wandstärke von bspw. 40µm und wird im warmen Zustand mit sich selbst kaschiert, das heißt zusammengefaltet. Anschließend werden die Ränder abgeschnitten, so daß eine Doppelschicht von 2 x 40µm Gesamtdicke entsteht. Diese Art der Fertigung führt dazu, daß beide Folienschichten gleich dick sind und aus dem gleichen Material bestehen. Die beiden Folien haften durch Adhäsion aneinander.

Vorteilhaft ist es, wenn das spaltbare Material einen Vliesstoff aufweist. Ein Vliesstoff bietet den Vorteil, daß er teilbar ist, wenn die beiden Seiten des Vliesstoffes auseinandergezogen werden. Nach Auftrennung des Vliesstoffes entstehen zwei Flächen mit textilem Charakter, die vorzugsweise sogar weich und flauschig ausgebildet sein können.

Eine Ausführungsform der Erfindung sieht vor, daß der Vliesstoff partiell thermisch oder mit einem Binder verfestigt ist. Beispielsweise ein thermoverfestigtes Polypropylen/Spinnvlies wird auf einer oder beiden Seiten verfestigt, während der mittlere Bereich des Vlieses weniger verfestigt und damit leicht spaltbar ist. Die verfestigten Flächen erleichtern die Beschichtung mit Klebstoff, die notwendig ist, um das Windelverschlußband an der Windel zu befestigen. Eine günstige Alternative zum Vliesstoff liegt darin, daß das spaltbare Material ein coextrudiertes Folienmaterial aufweist. Derartige Materialien werden zusammen extrudiert und haften ohne Klebstoff durch Adhäsion bzw. Cohäsion aneinander.

Vorteilhaft ist es, wenn der dritte Bereich zwischen dem ersten und dem zweiten Bereich angeordnet ist. Diese Anordnung erlaubt es, einerseits den zweiten Bereich bei der Herstellung und Verarbeitung der Windel fest an der Windel zu halten und andererseits bei der Verwendung der Windel durch ein Ziehen am zweiten Bereich das spaltbare Material aufzuspalten. Die hierfür erforderliche Kraft sollte im Bereich von ca. 0,1 - 10 N/inch, vorzugsweise 2,5 - 7 N/inch liegen.

Bei der Verwendung eines Trägermaterials wird vorgeschlagen, daß das spaltbare Material flächig mit dem Trägermaterial verklebt ist. Dadurch wird erreicht, daß sich bei der Verwendung der Windel das spaltbare Material tatsächlich aufspaltet und nicht vom Trägermaterial ablöst.

Um ein einfaches Auf- und Abrollen der ungeschnittenen Windelverschlußband-Rollenware sicherzustellen, weist der zweite und der dritte Bereich vorzugsweise im wesentlichen dieselbe Dicke auf. Dadurch erhält die Rollenware eine relativ breite ebene Fläche, die das Handling der Rollenware erleichtert.

Vorzugsweise weist der zweite Bereich einen mechanischen Verschlußteil auf. Derartige mechanische Windelverschlußsysteme bestehen beispielsweise aus einem Haken- oder Pilzkopfmaterial, das als männliches Teil des mechanischen Verschlußsystems auf einem weiblichen Teil an der Windel, wie beispielsweise auf der Wirkware oder einem Frontaltape auf Nonwoven-Basis befestigbar ist. Auch das Fixieren des männlichen Teils direkt auf der stoffähnlichen, faserigen (clothlike) Windelaußenhaut ist empfehlenswert. Verständlicherweise kann auch der männliche Teil auf der Windel und der weibliche Teil am Windelverschlußband befestigt sein.

Die gestellte Aufgabe wird auch mit einer Windel gelöst, die ein derartiges Windelverschlußband aufweist und bei der die eine Seite des spaltbaren Materials an der Innenseite der Windel befestigt ist. Dadurch wird der zweite, lösbar mit der Windel verbindbare Bereich des Windelverschlußbandes während der Windelherstellung fest an der Windel gehalten und bei der Verwendung der Windel kann der zweite Bereich leicht von der Windel weggezogen werden, indem sich das spaltbare Material aufspaltet. Sofern das spaltbare Material aus einem Vlies besteht, hat das Windelverschlußband gerade im Befestigungsbereich des Windelverschlußbandes an der Windel einen weichen, textilen Charakter und es entstehen keine Klebe-, oder Folienzonen, die mit der Haut des Windelträgers, das heißt, insbesondere des Babys in Berührung kommen könnten.

Vorteilhaft ist es weiterhin, wenn bei der erfindungsgemäßen Windel der zweite Bereich lösbar mit der Windelinnenseite verbunden ist und der erste Bereich an der Außenseite der Windel befestigt ist.

Ausführungsbeispiele der Erfindung sind in den Zeichnungen dargestellt und werden im folgenden näher beschrieben.

Es zeigt,
- Figur 1: einen Schnitt durch ein erfindungsgemäßes Windelverschlußband,
- Figur 2: einen Schnitt durch ein an einer Windel befestigtes Windelverschlußband,
- Figur 3: einen Schnitt durch ein an einer Windel befestigtes, zur Verwendung aufgerissenes Windelverschlußband,
- Figur 4: einen Schnitt durch ein Windelverschlußband mit teilweise entferntem spaltbarem Material,
- Figur 5: einen Schnitt durch ein Windelverschlußband ohne zusätzliches Trägermaterial,
- Figur 6: einen Schnitt durch ein Windelverschlußband mit Einschnitt und
- Fugur 7: einen Schnitt durch ein Windelverschlußband mit zusätzlicher Folie.

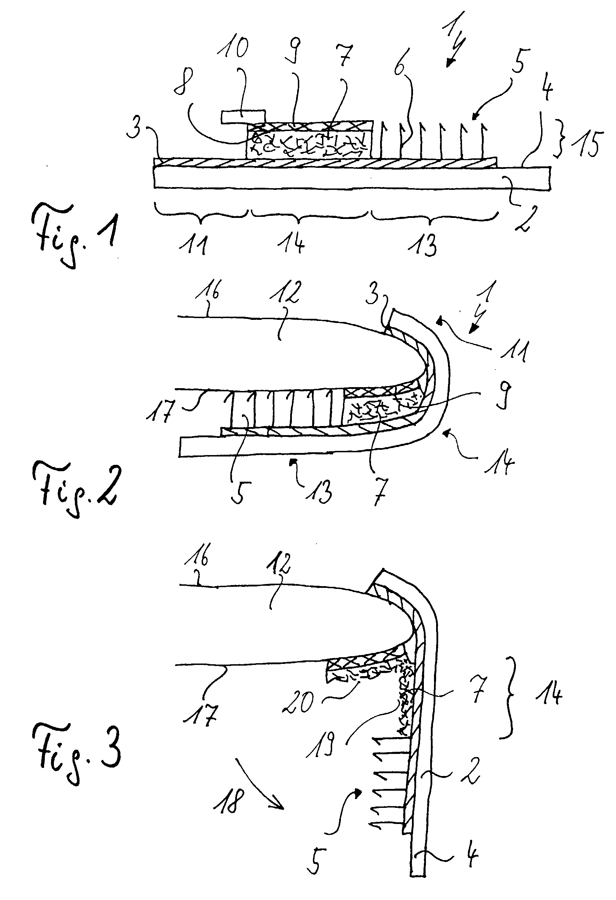

Das in Figur 1 dargestellte Windelverschlußband 1 hat ein Trägermaterial 2, das im vorliegendem Fall eine Folie ist, aber auch aus einem Vlies- oder einem Papiermaterial bestehen kann. Auf diesem Trägermaterial 2 ist eine Klebezone 3 aufgebracht, die sich über die gesamte obere Seite des Trägermaterials 2 mit Ausnahme eines Anfaßbereiches 4 erstreckt. Im Anschluß an den Anfaßbereich 4 ist auf der Klebezone 3 ein mechanischer Verschlußteil 5 vorgesehen, der sich über einen Teil der Klebezone 3 erstreckt. Dieser mechanische Verschlußteil ist ein sogenannter männlicher Verschlußteil, der aus vielen eng nebeneinander angeordneten Haken 6 besteht. An diesen mechanischen Verschlußteil 5 schließt sich auf der dem Anfaßbereich 4 gegenüberliegenden Seite eine Schicht aus einem spaltbarem Material 7 an, das ebenfalls an der Klebezone 3 befestigt ist. Dieses spaltbare Material besteht im vorliegendem Fall aus einem thermoverfestigten Polypropylenspinnvlies mit einem Flächengewicht zwischen 40 und 130 g/m², vorzugweise etwa 80 g/m². Die der Klebezone 3 gegenüberliegende Seite 8 des spaltbaren Materials 7 weist eine Klebstoffbeschichtung 9 auf, an der ein Kaschierband 10 befestigt ist.

Die Funktion des spaltbaren Materials ist auch dann gegeben, wenn das spaltbare Material 7 deutlich dünner ist als das Hakenmaterial am mechanischen Verschlußteil 5. In diesem Fall ist es jedoch vorteilhaft, wenn die Klebezone 9 einen Mindestabstand vom Hakenmaterial aufweist, um eine Berührung zwischen Haken und Klebezone zu vermeiden.

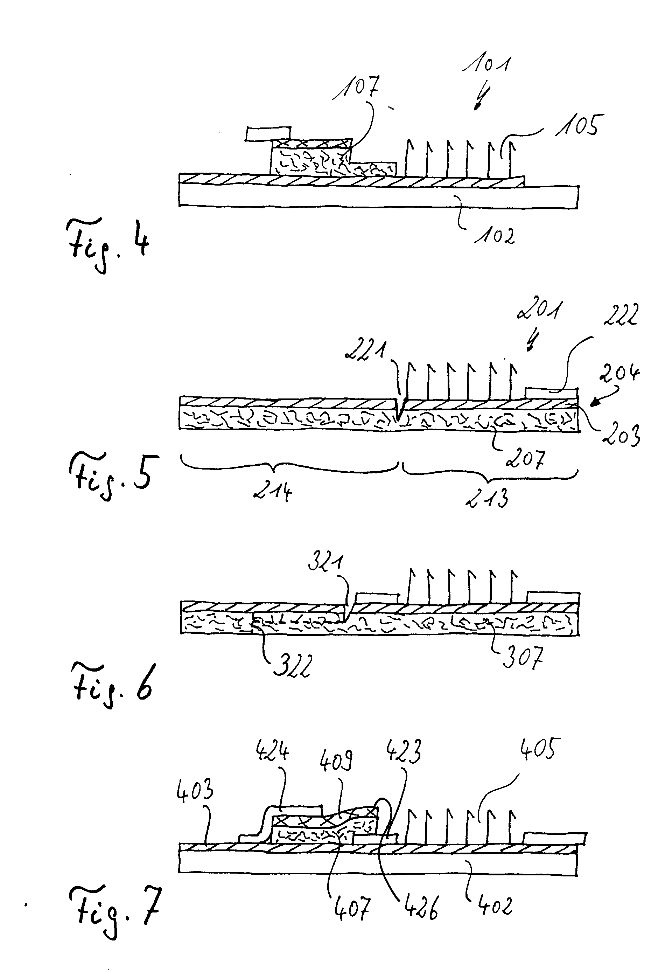

Das Windelverschlußband 1 hat somit einen ersten Bereich 11, der über die Klebezone 3 an einer Windel 12 (vergleiche Figur 2) befestigbar ist und einen zweiten Bereich 13, der lösbar mittels des mechanischen Verschlußteiles 5 mit einer Windel 12 verbindbar ist. Zwischen dem ersten Bereich 11 und dem zweiten Bereich 13 ist ein dritter Bereich 14, der über das spaltbare Material 7 und die Klebstoffbeschichtung 9 an einer Windel 12 befestigbar ist.

Das spaltbare Material 7 besteht aus einem Vliesstoff, der in seiner Dicke so gewählt ist, daß er in etwa der Dicke 15 des mechanischen Verschlußteils 5 entspricht. Dies erleichtert das Aufrollen des Windelverschlußbandes 1 in einer Richtung senkrecht zur Blattebene der Figur 1.

Das in Figur 2 gezeigte Windelverschlußband 1 hat einen ersten Bereich 11, der mittels der Klebezone 3 an der Außenseite 16 der Windel 12 befestigt ist. Das Windelverschlußband 1 ist derart um die Windel 12 herumgelegt, daß der zweite Bereich 13 mit dem mechanischen Verschlußteil 5 an der Innenseite 17 der Windel 12 anliegt. Der dazwischen liegende dritte Bereich 14 mit dem spaltbaren Material 7 ist über die Klebstoffbeschichtung 9 mit der Innenseite 17 der Windel 12 verklebt.

Während des Anlegens der Windel 12 wird das Trägermaterial 2, wie in Figur 3 gezeigt, von der Innenseite 17 der Windel 12 in Richtung des Pfeiles 18 weggezogen. Hierzu wird das Trägermaterial am Anfaßbereich 4 gegriffen und soweit in Richtung des Pfeiles 18 bewegt, bis das spaltbare Material 7 im dritten Bereich 14 aufgespalten ist. Der mechanische Verschlußteil 5 kann nun einfach am anderen Windelbereich (nicht gezeigt) befestigt werden und die zwei aufgerissenen Innenseite 19 und 20 des spaltbaren Materials 7 bilden eine weiche, textilartige Oberfläche, die ohne Probleme zu verursachen auch in Hautkontakt mit dem Windelträger treten kann und optisch gut zu einer Windel mit textiler Außenhaut paßt.

Das in Figur 4 gezeigte Windelverschlußband 101 ist im wesentlichen wie das in Figur 1 gezeigte Windelverschlußband 1 aufgebaut. Das spaltbare Material 107 ist aber auf der dem Trägermaterial 102 abgewandten Seite in der Nähe des Verschlußteils 105 während der Verschlußtapeherstellung zum Teil entfernt worden. Dadurch wird ein sicheres Aufspalten des spaltbaren Materials 107 während der Anwendung gewährleistet.

Figur 5 zeigt ein Windelverschlußband 201, bei dem das spaltbare Material 207 als Träger dient. Ein zusätzliches Trägermaterial ist dadurch nicht notwendig. Zwischen einem zweiten Bereich 213 und einem dritten Bereich 214 ist eine Einkerbung, oder eine Perforation 221 angebracht, die bei der Anwendung des Windelverschlußbandes 201 für ein Aufspalten des Materials 207 an der richtigen Stelle sorgt. Die Klebezone 203 verläuft bis in den Anfaßbereich 204 und ist im Anfaßbereich 204 mit einer Abdeckfolie 222 abgedeckt.

Auch bei dem Ausführungsbeispiel nach Figur 6 dient das spaltbare Material 307 als Träger. Ein Einschnitt, eine Einkerbung oder eine Perforation 321 gibt die Stelle an, von der beginnend das Material sich aufspalten soll und eine Verschweißung (z. B. mit Ultraschall) oder Verklebung 322 gibt die Stelle an, an der die Aufspaltung beendet werden soll.

Figur 7 zeigt ein weiteres Ausführungsbeispiel, bei dem zwischen dem mechanischen Verschlußteil 405 und dem spaltbaren Material 407 eine Folie 423 die Klebezone 403 abdeckt. Außerdem ist ein Kaschierband 424 vorgesehen, das einen Teil der Klebestoffbeschichtung 409 des spaltbaren Materials 407 und einen Teil der auf dem Trägermaterial 402 angebrachten Klebezone 403 abdeckt. Die Folie 423 sorgt dafür, daß das spaltbare Material 407 sicher mit der Windelinnenseite verklebt werden kann; auch wenn das mechanische Verschlußteil 405 deutlich dicker ist, als das spaltbare Material 407. Um eine sichere Spaltung zu gewährleisten, muß das spaltbare Material 407 bereits an der zum mechanischen Verschlußteil 405 weisenden Kante 426 fest mit der Windelinnenseite verklebt sein.

## Patentansprüche

1. Windelverschlußband (1) mit einem ersten Bereich (11), der an einer Windel (12) befestigbar ist, und einem zweiten Bereich (13), der lösbar mit der Windel (12) verbindbar ist, ***gekennzeichnet durch*** einen in der Nähe des zweiten Bereiches (13) angeordneten dritten Bereich (14), der ein spaltbares Material (7) aufweist, dessen eine Seite (8) an der Windel (12) befestigbar ist.

2. Windelverschlußband nach Anspruch 1, ***dadurch gekennzeichnet, da*ß** das spaltbare Material (7) einen Vliesstoff aufweist.

3. Windelverschlußband nach Anspruch 2, ***dadurch gekennzeichnet, daß*** der Vliesstoff thermisch oder mit einem Binder verfestigt ist.

4. Windelverschlußband nach Anspruch 1, ***dadurch gekennzeichnet, daß*** das spaltbare Material (7) ein coextrudiertes Folienmaterial aufweist.

5. Windelverschlußband nach Anspruch 1, ***dadurch gekennzeichnet, daß*** das spaltbare Material (7) zwei Hälften eines durch Blasextrusion hergestellten, mit sich selbst kaschierten Schlauches aufweist.

6. Windelverschlußband nach einem der vorhergehenden Ansprüche, ***dadurch gekennzeichnet, daß*** der dritte Bereich (14) zwischen dem ersten (11) und dem zweiten Bereich (13) angeordnet ist.

7. Windelverschlußband nach einem der vorhergehenden Ansprüche, ***dadurch gekennzeichnet, daß*** das spaltbare Material (7) flächig mit einem Trägermaterial (2) verklebt ist.

8. Windelverschlußband nach einem der vorhergehenden Ansprüche, ***dadurch gekennzeichnet, daß*** der zweite (13) und der dritte Bereich (14) im wesentlichen dieselbe Dicke (15) aufweisen.

9. Windelverschlußband nach einem der vorhergehenden Ansprüche, ***dadurch gekennzeichnet, daß*** der zweite Bereich (13) einen mechanischen Verschlußteil (5) aufweist.

10. Windel mit einem Windelverschlußband nach einem der Ansprüche 1 - 9, ***dadurch gekennzeichnet, daß*** die eine Seite (8) des spaltbaren Materials (7) an der Innenseite (17) der Windel (12) befestigt ist.

11. Windel nach Anspruch 10, ***dadurch gekennzeichnet, daß*** der zweite Bereich (13) lösbar mit der Innenseite (17) der Windel (12) verbunden ist.

12. Windel nach einem der Ansprüche 10 oder 11, ***dadurch gekennzeichnet, daß*** der erste Bereich (11) an der Außenseite (16) der Windel (12) befestigt ist.
